Europäisches Patentamt

European Patent Office

Office européen des brevets

(19)

(11) Numéro de publication: **0 317 451**
**A1**

## (12) DEMANDE DE BREVET EUROPEEN

(21) Numéro de dépôt: **88420380.3**

(22) Date de dépôt: **15.11.88**

(51) Int. Cl.4: **A 61 N 1/32**

(30) Priorité: **16.11.87 US 120920   17.03.88 US 169492**

(43) Date de publication de la demande:
**24.05.89   Bulletin   89/21**

(84) Etats contractants désignés:
**AT BE CH DE ES FR GB GR IT LI LU NL SE**

(71) Demandeur: **Klein, Oscar**
**401 Manatee Avenue West**
**Holmes Beach Florida 33510  (US)**

**Demartini, Richard J.**
**8380 Vimerton Road**
**206 Largo Florida 34641  (US)**

(72) Inventeur: **Klein, Oscar**
**401 Manatee Avenue West**
**Holmes Beach Florida 33510  (US)**

**Demartini, Richard J.**
**8380 Vimerton Road**
**206 Largo Florida 34641  (US)**

(74) Mandataire: **Dupuis, François**
**Cabinet Charras 3 Place de l'Hôtel-de-Ville BP 203**
**F-42005 St. Etienne Cédex 1  (FR)**

(54) Dispositif électronique amélioré générateur d'impulsions pour applicateur facial et méthode de fabrication.

(57)   Ce générateur est remarquable en ce qu'il comprend :
- un moyen applicateur tenu à la main (10) ayant une excitation sé-parée des surfaces (12-14) profilées généralement conductrices séparées par un contact de base central (28), les surfaces étant appliquées et frottées doucement sur une zone de la surface de la peau que l'on souhaite traiter,
- un moyen de circuit électronique générateur d'impulsions (40) ayant des bornes pour connexion à un courant de sortie aux surfaces respectives des surfaces conductrices disposées sur l'applicateur tenu à la main, et le courant de sortie ayant des caractéristiques de gain, fréquence de répétition, fréquence et amplitude et,
- un interrupteur monté sur un tableau de commande du moyen applicateur pour choisir une polarité du courant appliquée à la surface respective des surfaces conductrices.

FIG.1

EP 0 317 451 A1

## Description

**Dispositif Electronique amélioré générateur d'impulsions pour Applicateur Facial et Méthode de fabrication.**

### Historique de l'invention

### Domaine d'application de l'invention

Cette demande est une demande de continuation de notre demande de brevet en coinstance numéro d'ordre 06/120.920 déposée le 16 novembre 1987 et la totalité de l'exposé de cette demande est incorporée ici par référence.

L'invention concerne un dispositif électronique amélioré générateur d'impulsions pour applicateur facial utilisé dans l'application de liquides et/ou crèmes sur des parties de la peau du corps, et plus particulièrement l'invention concerne un circuit électronique générateur d'impulsions et une tête d'application mise au point pour utilisation avec son système d'électrodes pour aider à la pénétration d'un liquide ou d'une crème, comme Retin-A, décrit ci-après, ou autres liquides ou crèmes similaires, dans les pores de la peau, le dispositif comprenant un sélecteur pour permettre la sélection de deux modes de polarité réversible, de telle sorte qu'un mode de fonctionnement cause la dilatation des pores, et un second mode provoque une douce constriction des pores.

Le générateur de signaux de tension amélioré pour application des courants électriques choisis à un applicateur facial est relié à un applicateur tenu à la main ayant des surfaces conductrices réparties sur les surfaces généralement incurvées ou profilées ou ellipsoïdales de celui-ci et séparées par une partie de contact de base de telle sorte que les surfaces puissent être doucement appliquées et frottées sur une zone de la surface de la peau que l'on souhaite traiter, un circuit électronique générateur d'impulsions ayant des bornes pour raccordement à un courant de sortie aux surfaces conductrices respectives disposées sur l'applicateur tenu à la main, et le courant de sortie ayant des caractéristiques de gain, fréquence de répétition, fréquence et amplitude, et un sélecteur monté sur un tableau de commande de l'applicateur pour choisir une polarité du courant appliquée à la surface respective des surfaces de conduction.

L'invention concerne un appareil de traitement de la peau et la méthode pour son utilisation et application à la peau de l'utilisateur qui se propose de permettre à l'utilisateur de paraître plus jeune sans avoir à subir de douleur et de frais de chirurgie, de telle sorte que l'on puisse en conséquence subir l'application et l'utilisation de la technique connue comme ELECTRONIC FACE-LIFT®. Le traitement est réalisé avec un appareil tenu à la main, une machine ou un applicateur à pile ayant une préparation tonifiante et une fine crème antiride, comme Retin-A ou similaire, et le tout étant réalisé en l'espace de quelques minutes dans l'intimité, chez soi. Les méthodes des techniques antérieures englobent les traitements électroniques de lifting facial assurés par des professionnels dans un salon cosmétique onéreux, mais par l'utilisation de l'appareil et de la méthode du système de tension de rajeunissement, le traitement peut être administré chez soi, au bureau ou à tout endroit en quelques minutes.

L'applicateur a une tête d'application amovible de forme ellipsoïde construite et prévue pour atteindre toutes les zones possibles du visage y compris certaines zones des yeux et lèvres difficiles à atteindre, et en raison de la construction de l'applicateur amovible, le nettoyage est facile, simple et instantané, ainsi la tête peut être simplement extraite et nettoyée. La tête de l'applicateur est configurée en forme de pierre précieuse pour en faciliter la prise pour en faciliter la manutention au maximum. Un tableau de commande encastré protège les sélecteurs de gain et mode. Les commandes du sélecteur de gain de sortie comprennent cinq positions de sélection pour un réglage optimal. Est également comprise dans le sélecteur de mode la sélection des fonctions de PREP ou SET de l'applicateur. Un commutateur MARCHE/ARRET automatique sensible à la pression qui est incorporé dans la tête de l'applicateur est actionné en appuyant légèrement la tête de l'applicateur sur les surfaces de traitement choisies.

Une caractéristique de l'invention concerne de plus le circuit électronique générateur d'impulsions pour appliquer des amplitudes d'intensité variable ou réglable, des fréquences d'impulsion et des polarités variables ou réglables, à un circuit de temporisation ayant au moins deux portes "NON-OU" reliées en une relation de circuit de verrouillage, et produisant des impulsions de sortie à une borne, un circuit résistance-capacité contrôlant le circuit de temporisation pour assurer le réglage pour faire varier la largeur des impulsions de sortie à une borne, un amplificateur réagissant aux impulsions de sortie de largeur réglée et ayant une boucle de réaction pour contrôler le gain de l'amplificateur et ayant une résistance à une sortie de celui-ci de telle sorte que la résistance se comporte comme une résistance d'isolement, un amplificateur limite réagissant à la sortie sur la résistance d'isolement qui assure des caractéristiques de sécurité pour protéger le circuit électronique générateur d'impulsions contre le dépassement d'une sortie nominale, une alimentation électrique fournissant le courant au circuit de temporisation et d'un côté d'un enroulement primaire d'un transformateur, l'autre côté de celui-ci étant relié à la sortie de l'amplificateur limite, un enroulement secondiare du transformateur ayant une diode Zener accouplée à l'enroulement secondaire pour limiter la tension et éliminer les pointes de courant et de tension négatives, un interrupteur comme un commutateur bipolaire à deux directions (DPDT) accouplé à la diode Zener utilisé pour permuter la polarité du circuit électronique générateur d'impulsions, et un affichage comprenant un circuit à diodes électroluminescentes réagissant au circuit résistance-capacité pour afficher la puissance et la fréquence générées par le circuit électronique générateur d'impulsions.

L'invention concerne de plus un dispositif assurant un générateur de signaux de tension comme un ensemble de générateur électronique d'impulsions et électrodes spécialement mis au point dans le but d'augmenter la pénétration d'un liquide ou d'une crème, comme Retin-A, dans les pores de la peau. L'application de ce circuit et de cet appareil est un moyen plus sûr et plus efficace pour induire à la fois une vasco dilatation et une vasco-constriction avec une stimulation pour électrothérapie. L'appareil est fait et construit pour être tenu à la main et pour frotter doucement sur une surface de la peau que l'utilisateur souhaite affecter et traiter avec le médicament donné. La première phase consiste à sélectionner le mode d'utilisation de l'appareil. En choisissant un mode PREP., où de grosses parties extérieures de la tête de l'applicateur sont chargées négativement par rapport à la bande de contact intérieure disposée dans et passant par une partie centrale de la tête de l'applicateur. Théoriquement, ce mode PREP. a tendance à ouvrir les pores dans les zones traitées. Le signal utilisé pour réaliser la première opération est un signal semi-carré de 16 volts à 33 volts, 80-120 Hz, avec une largeur d'impulsion de 1 ms. La vitesse de l'impulsion crée un courant pseudo-galvanique et en même temps, la stimulation de l'impulsion fait vibrer les surfaces de la peau à traiter. Cela permet au liquide d'être absorbé en profondeur dans la peau dans un délai de 2,5 minutes, et en même temps, les vibrations aident le liquide à enduire l'intérieur des pores ouverts avec le liquide ou la crème jusqu'aux niveaux les plus profonds. Enfin, le régénérateur de tension est utilisé dans l'autre mode, identifié comme mode SET, qui est utilisé pour fermer doucement les pores ouverts, les impulsions positives créant un courant pseudo-galvanique qui, en conjonction avec l'effet de pulsation, ferme les pores sans la tension désagréable de la peau, produite par d'autres systèmes. Cette phase dure environ 1,3 minutes. Ainsi, tout le processus demande environ dix minutes ou moins pour réaliser un lifting rajeunissant réparateur conforme à l'invention. L'invention englobe également les nouvelles phases de la méthode de construction, en utilisant et employant un dispositif électronique amélioré générateur d'impulsions pour applicateur facial tel qu'il est plus particulièrement décrit ci-après.

### Description de la Technique Antérieure

Divers dispositifs de la technique antérieure semblables à un dispositif électronique générateur d'impulsions pour applicateurs faciaux de l'invention, et similaire, ainsi qu'un appareil et méthode pour leur construction en général, sont connus et des exemples de la technique antérieure américaine sont les suivants:

| | |
|---|---|
| 3.107.672 | Hofmann |
| 3.424.165 | Moss |
| 3.601.126 | Estes |
| 4.033.356 | Hara |
| 4.141.359 | Jacobsen et al |
| 4.301.794 | Tapper |
| 4.340.047 | Tapper |
| 4.406.658 | Lattin et al |
| 4.532.938 | Carlisle |
| 4.619.252 | Ibbot |
| 4.655.232 | Ficke |

Robin Marantz Henig, Physicians as Beauticians-The War on Wrinkles, The Washington Post - Section Santé, pages 12 et 14, 23 février 1988.

Abigail Trafford, The New Medical Quick Fix and the Fountain of Youth, The Washington Post - Section Santé, page 14, 23 février 1988.

Nancy Konesko, Retin-A is in demand, Bradenton(FL),Herald, pages C4, C7, 1988.

Les dispositifs de fontophorèse de Lattin et al., Tapper et Jacobsen et al sont considérés comme un appareil conçu pour améliorer l'absorption d'un médicament par la peau, de plus celui de Lattin et al. révèle la caractéristique de polarité réversible. Ficke et Carlisle, Hara, Moss et Hofmann sont des exemples d'applicateurs électriques tenus à la main conçus pour un usage médical. La plupart sont utilisés pour la stimulation des muscles et non pour aider à l'absorption de médicament par la peau. Ibbott et Estes présentent simplement des exemples de traitements médicaux électriques.

L'article de Hening se réfère au Dr Albert Kigman, professeur de dermatologie à l'Université de Pennsylvanie et l'auteur de la découverte en 1971 de Retin-A ; ce Retin-A est annoncé le 21 janvier 1988 essentiellement comme étant une crème stoppant les rides, dont une forme est déclarée être fabriquée par Ortho Pharmaceuticals of Raritan, NJ et que JAMA du 22 janvier 1988 a semblé confirmer. Trafford et Konesko discutent de plus des aspects de Retin-A.

Ces brevets ou les utilisations antérieures connues enseignent et révèlent divers types de dispositifs électroniques générateurs d'impulsions pour applicateur corporel de différentes sortes et de diverses fabrications et autres ainsi que les méthodes de leur construction, mais aucun d'eux pris individuellement ou collectivement ne révèle les détails spécifiques de la combinaison de l'invention de façon à retomber sur les revendications de la présente invention.

### Résumé de l'Invention

Un objet, avantage et caractéristique de l'invention est de fournir un nouveau générateur de signaux de tension amélioré pour application de courants électriques sélectionnés à un dispositif applicateur facial consistant en un applicateur tenu à la main ayant des surfaces conductrices généralement profilées ou ellipsoïdales et les surfaces étant appliquées et frottées doucement sur une zone de la surface de la peau que l'on souhaite traiter, un circuit électronique générateur d'impulsions ayant des bornes pour raccordement à un courant de sortie aux zones respectives des surfaces conductrices disposées sur l'applicateur tenu à la main, et le courant de sortie ayant des caractéristiques de gain, fréquence de répétition, fréquence et amplitude, et un commutateur monté sur un tableau de commande de l'applicateur pour sélectionner une polarité du courant appliquée à la zone respective des surfaces conductrices.

Un autre avantage, objet et caractéristique de l'invention est de fournir un circuit électronique générateur d'impulsions pour appliquer des amplitudes d'intensité variable ou réglable, des fréquences d'impulsion et des polarités variables ou réglables, à un circuit de temporisation ayant au moins deux portes NON-OU" reliées en une relation de circuit de verrouillage, et produisant des impulsions de sortie à une borne, un circuit résistance-capacité contrôlant le circuit de temporisation pour assurer le réglage pour faire varier la largeur des impulsions de sortie à une borne, un amplificateur réagissant aux impulsions de sortie de largeur réglée et ayant une boucle de réaction pour contrôler le gain de l'amplificateur et ayant une résistance à une sortie de celui-ci de telle sorte que la résistance se comporte comme une résistance d'isolement, un amplificateur limite réagissant à la sortie sur la résistance d'isolement qui assure des caractéristiques de sécurité pour protéger le circuit électronique générateur d'impulsions contre le dépassement d'une sortie nominale, une alimentation électrique fournissant le courant au circuit de temporisation et d'un côté d'un enroulement primaire d'un transformateur, dont l'autre côté est relié à la sortie de l'amplificateur limite, un enroulement secondaire du transformateur ayant une diode Zener accouplée à l'enroulement secondaire pour limiter la tension et éliminer les pointes de courant et de tension négatives, un sélecteur qui est un commutateur bipolaire à deux directions (DPDT) accouplé à la diode Zener utiliser pour permuter la polarité du circuit électronique générateur d'impulsions, et un affichage comprenant un circuit à diodes électroluminescentes (LED) réagissant au circuit résistance-capacité pour afficher la puissance et la fréquence générées par le circuit électronique générateur d'impulsions.

Un autre objet de l'invention est de plus orienté vers un dispositif ou appareil fait pour être tenu à la main et pour frotter doucement sur la zone de la peau que l'utilisateur souhaite traiter. Il y a une bande de contact sur le côté de l'appareil, de telle sorte que, lorsque l'appareil est tenu, un contact soit établi entre la machine et la main, ce qui fournit à un pôle du courant provenant de la machine un point de référence pour diriger la charge de l'applicateur vers la zone de la peau. Ceci détermine les surfaces de contact sur l'applicateur qui permettent à une charge polarisée contrôlée sélectionnable à l'applicateur d'être ainsi appliquée à la surface de la peau à côté de l'appareil. Un commutateur bipolaire à deux directions (DPDT) monté sur l'appareil applicateur détermine la polarité appliquée. Le gain du circuit électronique peut se régler à partir d'une commande de gain montée sur l'applicateur, et un commutateur MARCHE/ARRET est un commutateur instantané également monté sur l'applicateur et garantit à l'utilisateur le maintien des surfaces de contact en relation ferme suffisante pour une bonne application du système à la surface faciale.

Egalement un objet de l'invention est de fournir un circuit électronique générateur d'impulsions pour appliquer des amplitudes d'intensité variable ou réglable, des fréquences d'impulsion et des polarités variables ou réglables, à un applicateur tenu à la main comprenant un circuit de temporisation ayant au moins deux portes "NON-OU" reliées en une relation de circuit de verrouillage, et produisant des impulsions de sortie à une borne, un circuit résistance-capacité contrôlant le circuit de temporisation pour assurer le réglage pour faire varier la largeur des impulsions de sortie à une borne, un amplificateur réagissant aux impulsions de sortie de largeur réglée et ayant une boucle de réaction pour contrôler le gain de l'amplificateur et ayant une résistance à une sortie de celui-ci de telle sorte que la résistance se comporte comme une résistance d'isolement, un amplificateur limite réagissant à la sortie sur la résistance d'isolement qui assure des caractéristiques de sécurité pour protéger le circuit électronique générateur d'impulsions contre le dépassement d'une sortie nominale, une alimentation électrique fournissant le courant au circuit de temporisation et d'un côté d'un enroulement primaire d'un transformateur, dont l'autre côté est relié à la sortie de l'amplificateur limite, un enroulement secondaire du transformateur ayant une diode Zener accouplée à l'enroulement secondaire pour limiter la tension et éliminer les pointes de courant et de tension négatives, un commutateur bipolaire à deux directions (DPDT) accouplé à la diode Zener utilisé pour permuter la polarité du circuit électronique générateur d'impulsions aux surfaces, et un affichage comprenant un circuit à diodes électroluminescentes (LED) réagissant au circuit résistance-capacité pour afficher la puissance et la fréquence générées par le circuit électronique générateur d'impulsions.

La théorie d'application pour l'application du générateur de signaux de tension doit être examinée. Tout d'abord, l'appareil ou applicateur appliqué sur la surface traitée est utilisé en mode négatif lorsqu'une charge sur une surface ou des surfaces sur la tête de l'applicateur est négative par rapport à la bande de contact d'un côté de l'applicateur tenu à la main. On a constaté que ce mode ouvre les pores de la zone traitée et le signal utilisé réalise une première opération de signaux semi-carrés de -15 -à -33 volts, 60-120 Hz, avec une largeur d'impulsion de 1 ms. L'action des impulsions appliquées à la zone affectée ou traitée engendre la création d'un courant pseudo-galvanique alors que, en même temps, l'effet de pulsation provoque la vibration de la zone traitée de la peau, ce qui permet aux liquides ou aux crèmes disposées sur la zone traitée d'être absorbés en profondeur dans la zone de la peau dans un délai d'environ 3 à 5 minutes, et, en même temps, les vibrations aident les liquides ou crèmes à enduire l'intérieur des pores ouverts. Ceci tend à assurer que les liquides et crèmes atteignent même des profondeurs très grandes des pores. Deuxièmement, le générateur de signaux de tension est utilisé en mode positif pour fermer doucement les pores à l'état ouvert. Les impulsions positives tendent à créer un courant pseudo-galvanique qui, en liaison avec l'effet de pulsation, ferme les pores sans tirer la peau traitée, phase qui dure environ trois minutes.

Ces objets et avantages avec d'autres qui apparaîtront ultérieurement résident dans les détails du procédé et de sa mise en oeuvre comme cela sera défini et revendiqué plus complètement par la suite, en ajoutant la référence aux plans d'accompagnement qui en font partie, où des chiffres identiques se réfèrent à des pièces identiques partout.

Description des Différentes Vues des Plans

La Figure 1 est une vue de face de l'intérieur d'un applicateur facial amélioré à signaux de tension à l'état ouvert et illustrant une installation type de l'applicateur conformément à une réalisation préférée et au meilleur mode de la présente invention.

La Figure 2 est une vue latérale prise suivant les lignes 2-2 de la Figure 1 et englobant les concepts de l'invention.

La Figure 3 est une vue inférieure de l'applicateur présentant les surfaces de contact de l'applicateur séparées par un centre diélectrique.

La Figure 4 est une vue de face d'un extérieur de l'applicateur.

La Figure 5 est une vue réduite d'une partie de face de l'applicateur prise suivant les lignes 5-5 de la Figure 2 et englobant les concepts de l'invention.

La Figure 6 est une disposition type et préférée représentant un schéma de circuit du générateur amélioré de signaux de tension pour l'applicateur.

### Description d'une Réalisation Préférée de l'Invention.

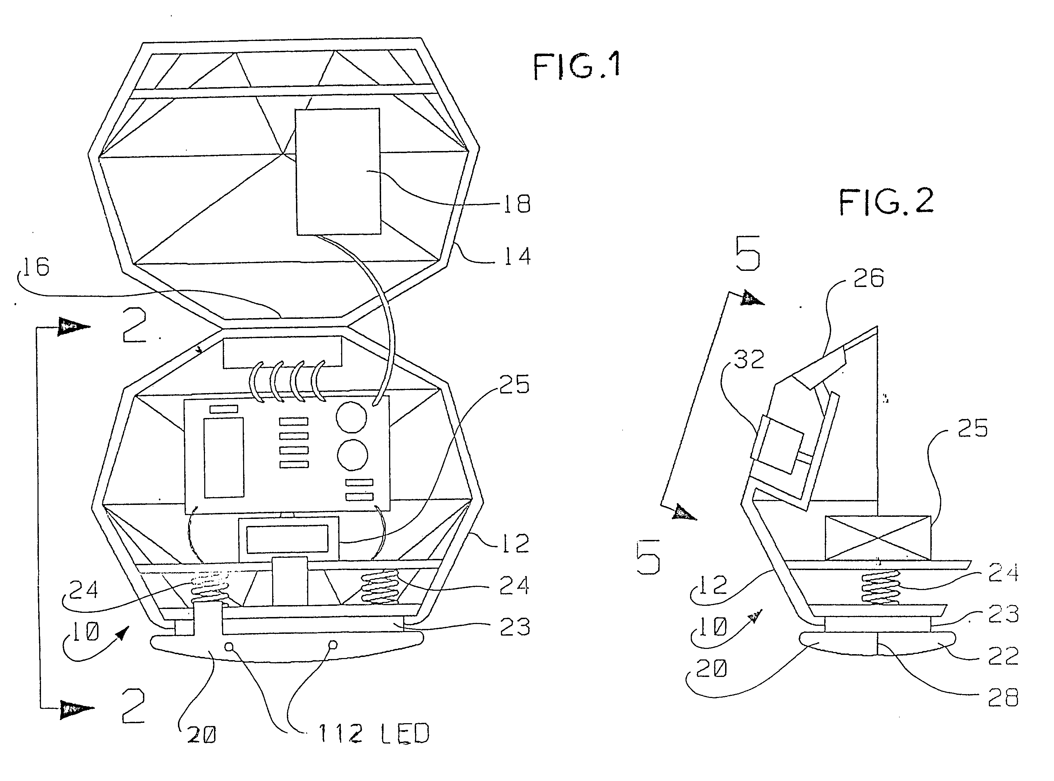

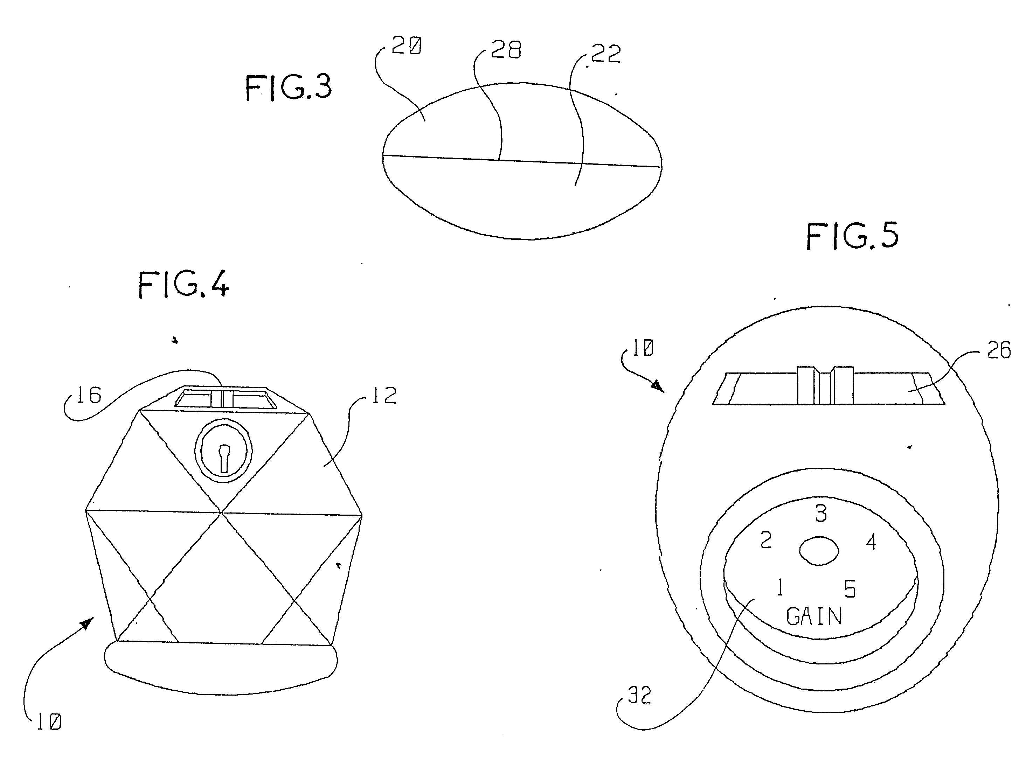

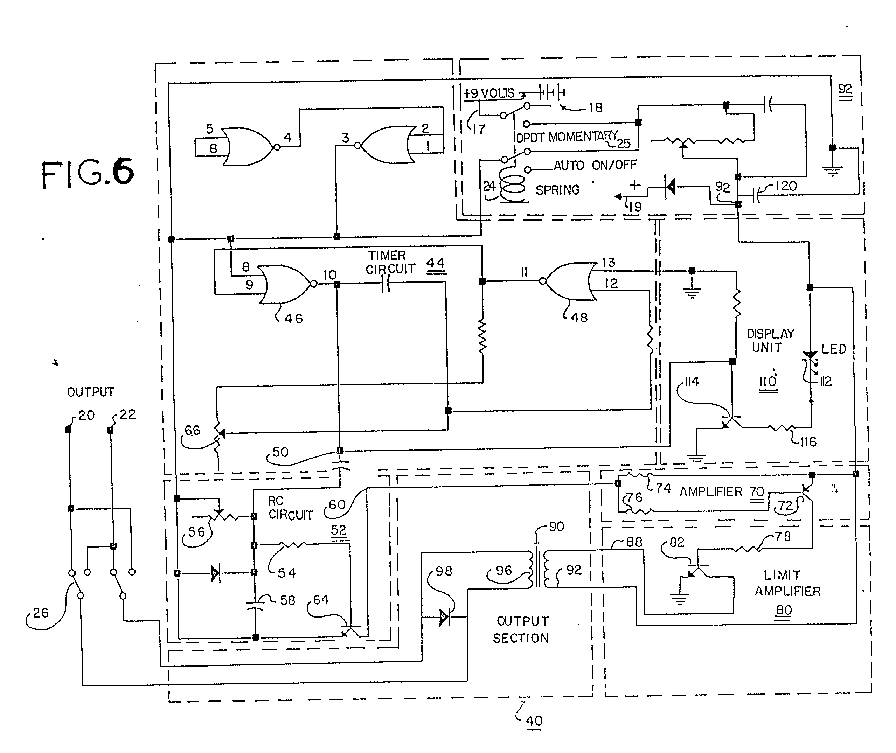

En se référant maintenant aux plans, il est représenté aux Figures 1-5 un applicateur facial tenu à la main (10) construit et conçu pour posséder des boîtiers en deux éléments (12, 14) qui sont montés avec une articulation ou élément de pivot (16), et ayant, comme le montre particulièrement la Figure 3, au moins deux surfaces curvilinéaires conductrices ou surfaces ellipsoïdales (20, 22) séparées par un contact de base central (28) monté sur une base de support ou entretoise (23) contraintes à dépasser vers le bas par des ressorts de tension (24), mais lorsqu'elles sont poussées vers le haut, elles tendent à faire mettre le commutateur automatique MARCHE/ARRET (25) sur une position fermée, vers le bas, comme pourrait le montrer le circuit de la Figure 6. Les surfaces ellipsoïdales (20, 22) doivent être doucement appliquées et frottées sur une zone de la peau de l'utilisateur qu'il souhaite traiter, et ayant au moins les deux surfaces conductrices (20, 22).

Les boîtiers (12, 14) peuvent être construits en matières plastiques ou en matière élastomère, et la surface sur les boîtiers (12, 14) peut comprendre une configuration ayant la forme générale d'une pierre précieuse pour faciliter la manipulation par l'utilisateur et cela peut être des têtes d'applicateurs ellipsoïdales en acier inoxydable, séparées par le contact de base central (28). Un sélecteur de polarité ou commutateur bipolaire à deux directions (26) est monté sur un tableau de commande ou le côté de l'applicateur (10) et sert à choisir la polarité du courant appliqué à la surface respective des surfaces conductrices (20, 22) ou contact de base central (28). Sur l'un ou l'autre côté des boîtiers (12, 14) de l'applicateur (10) se trouve un sélecteur de mode PREP/SET bipolaire à deux directions (DPDT) (26), un interrupteur de commande du gain de sortie (32) qui peut être accouplé à une ou plusieurs résistances variables (56, 66).

L'applicateur (10) peut de préférence inclure une pluralité, ou une série de surfaces conductrices (20, 22), certaines reliés pour excitation d'une polarité, et le contact de base central relié pour excitation de l'autre polarité.

Soit à l'extérieur, soit de préférence à l'intérieur de l'applicateur (10) se trouve un circuit électronique générateur d'impulsions (40) représenté à la Figure 6 et ayant des bornes sur le sélecteur bipolaire à deux directions (DPDT) (26) pour raccordement à un courant de sortie aux surfaces respectives des surfaces conductrices (20, 22), prévues sur l'applicateur (10) tenu à la main, et le courant de sortie ayant des commandes des caractéristiques de gain, fréquence de répétition, fréquence et amplitude.

La Figure 6 représente le circuit électronique générateur d'impulsions (40) ayant un circuit de temporisation (44) comprenant au moins deux portes "NON-OU" (46,48) reliées suivant une relation de circuit de verrouillage et produisant des impulsions de sortie à une borne (50) de celui-ci. Un réseau résistance-capacité (52) ayant une résistance (54), résistance variable (56) et capacité (58), commande le circuit de temporisation (44) pour assurer le réglage pour faire varier la largeur des impulsions de sortie à une borne (60) de celui-ci. Le transistor (64) est relié à la résistance (54) et capacité (58) de telle sorte que le transistor (64) commande ou permettre à une impulsion de passer à la vitesse de consigne contrôlée par la résistance (66), et cette impulsion est appliquée à l'amplificateur (70) comprenant le transistor (72), et les résistances (74, 76) qui sont reliées en une boucle de réaction au transistor (72) pour contrôler la sortie de gain de celui-ci.

La résistance (78) reliée électriquement à un amplificateur limite (80) se comporte comme une résistance d'isolement en envoyant une impulsion à l'amplificateur-transistor (82) utilisé comme phase de limitation pour la sortie de courant. En raison de la nature du transistor (82), il ne peut que délivrer une quantité minime de courant, même s'il est ouvert au gain total, ce qui constitue une caractéristique de sécurité pour protéger le circuit contre le dépassement des caractéristiques nominales de sortie à la dernière phase. La borne de sortie (88) de l'amplificateur limiteur (80) est reliée à un côté d'un transformateur élévateur (90) alors que l'autre côté est accouplé à une source d'alimentation (92) qui fournit également le courant au circuit de temporisation (44). La source d'alimentation (92) assure ainsi un circuit de commande complet.

Un enroulement secondaire (96) du transformateur (90) a une diode Zener (98) accouplée à l'enroulement secondaire (96) pour limiter la tension de sortie à 30 volts maximum et éliminer les points de courant et tension négatives à la phase de sortie finale. Le sélecteur (26) de préférence un commutateur bipolaire à deux directions (DPDT) est accouplé à la diode Zener (98) et utilisé sélectivement pour permuter la polarité du circuit électronique générateur d'impulsions (40).

Un ensemble de visualisation (110) englobant une diode électroluminescente (112) commandée à partir d'une impulsion de temporisation provenant de la borne (50) du circuit de temporisation (44) appliquée au transistor (114) et à la résistance (116) réagit au circuit résistance-capacité (52) pour afficher la puissance et la fréquence générées par le circuit électronique générateur d'impulsions (40).

La source d'alimentation (92)est une pile standard 9 volts qui a une capacité (120) shuntée pour prolonger d'une façon générale la longévité de la pile.

L'appareil de l'applicateur facial (10) de l'invention peut être construit et constitué par ses éléments constitutifs de telle sorte qu'il puisse être assemblé comme un kit ou sous la forme d'un kit.

Tout ce qui précède est considéré seulement comme une illustration des principes de l'invention. De plus, puisque de nombreuses modifications et de nombreux changements seront aisément apportés par des hommes de métier, il n'est pas souhaité limiter l'invention à la construction exacte et au fonctionnement illustrés et décrits, et en conséquence, toutes les modifications adéquates et équivalences peuvent être considérées comme entrant dans le cadre de l'invention.

## Revendications

-1- Générateur amélioré de signaux de tension pour application de courants électriques sélectionnés à un applicateur facial caractérisé en ce qu'il comprend :
- un moyen applicateur tenu à la main (10) ayant une excitation sé-parée des surfaces (12-14) profilées généralement conductrices séparées par un contact de base central (28), les surfaces étant appliquées et frottées doucement sur une zone de la surface de la peau que l'on souhaite traiter,

- un moyen de circuit électronique générateur d'impulsions (40) ayant des bornes pour connexion à un courant de sortie aux surfaces respectives des surfaces conductrices disposées sur l'applicateur tenu à la main, et le courant de sortie ayant des caractéristiques de gain, fréquence de répétition, fréquence et amplitude et,
- un interrupteur monté sur un tableau de commande du moyen applicateur pour choisir une polarité du courant appliquée à la surface respective des surfaces conductrices.

-2- Appareil selon la revendication 1 caractérisé en ce que le commutateur est un commutateur bipolaire à deux directions (DPDT) (26).

-3- Appareil selon la revendication 1 caractérisé en ce que les surfaces sont au moins au nombre de deux.

-4- Appareil selon la revendication 1 caractérisé en ce que le moyen de circuit électronique générateur d'impulsions comprend :
- un circuit de temporisation (44) ayant au moins deux portes (46-48) "NON-OU" reliées suivant une relation de circuit de verrouillage et produisant des impulsions de sortie à une borne (50),
- un moyen résistance-capacité (52) contrôlant le circuit de temporisation pour assurer le réglage pour faire varier la largeur des impulsions de sortie à une borne,
- un moyen amplificateur (70) réagissant aux impulsions de sortie à largeur réglée et ayant une boucle de réaction pour contrôler le gain de l'amplificateur et ayant une résistance à une sortie, de telle sorte que la résistance se comporte comme une résistance d'isolement,
- un amplificateur limite (80) réagissant à la sortie sur la résistance d'isolement assure les caractéristiques de sécurité pour protéger le moyen de circuit électronique générateur d'impulsion contre le dépassement d'une sortie nominale,
- un moyen d'alimentation fournissant le courant au circuit de temporisation (44) et un côté d'enroulement primaire d'un transformateur (90) dont l'autre côté est accouplé à la sortie de l'amplificateur limite,
- un enroulement secondaire (96) du transformateur ayant une diode Zener (98) accouplée à l'enroulement secondaire pour limiter la tension et éliminer les pointes négatives de courant et de tension,
- le sélecteur (26) étant un commutateur bipolaire à deux directions (DPDT) accouplé à la diode Zener et utilisé pour permuter la polarité du moyen circuit électronique générateur d'impulsions, et
- un moyen de visualisation (110) comprenant un moyen de circuit à diode électroluminescente (112) réagissant au moyen résistance-capacité pour afficher la puissance et la fréquence générées par le moyen de circuit électronique générateur d'impulsions.

-5- Appareil selon la revendication 1 caractérisé en ce que le moyen du circuit électronique générateur d'impulsions (40) comprend un moyen disposé sur l'applicateur tenu à la main qui assure le contrôle des caractéristiques de gain, fréquence de répétition, fréquence et amplitude.

-6- Appareil selon la revendication 1 caractérisé en ce que l'utilisation des paramètres du moyen de circuit électronique générateur d'impulsions assure l'ouverture en profondeur des pores de la peau permettant et favorisant une pénétration de liquide ou crème dans les pores.

-7- Appareil selon la revendication 1 caractérisé en ce que l'applicateur tendu à la main est une méthode plus sûre et plus efficace pour provoquer une vasco-dilatation en même temps qu'une stimulation pour électrothérapie.

-8- Appareil selon la revendication 1 caractérisé en ce que les surfaces sont généralement ellipsoïdales (20-22).

-9- Appareil selon la revendication 1 caractérisé en ce que la surface planaire a une série de surfaces conductives réparties sur celle-ci.

-10- Appareil selon la revendication 1 caractérisé en ce que la surface planaire est particulièrement adaptée au contact avec et frotte sur une partie du visage.

-11- Appareil selon la revendication 1 caractérisé en ce que l'applicateur est construit en matière plastique.

-12- Appareil selon la revendication 1 caractérisé en ce que l'applicateur est construit en matière élastomère.

-13- Méthode de fabrication et application de signaux électroniques de tension améliorés de courants électriques sélectionnés pour application à un applicateur facial caractérisées en ce qu'elles comprennent les phases de :

- génération d'un signal de tension par un moyen de circuit éléctronique générateur d'impulsions ayant des bornes pour branchement à un courant de sortie aux surfaces respectives des surfaces conductrices disposées sur l'applicateur tenu à la main, et le courant de sortie ayant des caractéristiques de gain, fréquence de répétition, fréquence et amplitude.

- application d'un moyen applicateur tenu à la main ayant des surfaces conductrices réparties sur une surface généralement planaire de celui-ci et à appliquer et frotter doucement sur une zone de la surface de la peau que l'on souhaite traiter.

- commutation à un mode négatif où la tête de l'applicateur est négative par rapport à la bande de contact sur un côté de l'applicateur tenu à la main, les constatations ayant montré que ce mode ouvre les pores de la zone traitée, le signal utilisé réalise une première opération de signaux semi-carrés de -15 à -33 volts, 60-120 Hz, avec une largeur d'impulsion de 1ms, et provoque la création d'un courant pseudo-galvanique tandis qu'en même temps l'effet de pulsation provoque la vibration de la zone de la peau traitée, ainsi

permettant aux liquides ou crèmes disposés sur la zone traitée d'être absorbés en profondeur dans la zone de la peau dans un délai d'environ 3-5 minutes, et en même temps, les vibrations aident les liquides ou crèmes à enduire l'intérieur des pores ouverts et à assurer que les liquides et crèmes parviennent même à de très grandes profondeurs des pores, et

commutation du générateur de signaux de tension à un mode positif pour fermer doucement les pores ouverts, de telle sorte que les impulsions positives tendent à créer un courant pseudo-galvanique qui, en liaison avec l'effet de pulsation, ferme les pores sans tirer la peau traitée, phase qui dure environ trois minutes.

FIG.1

FIG.2

EP 0 317 451 A1

FIG.3

20    28    22

FIG.5

FIG.4

16        12

10

10        26

3
2       4
1       5
GAIN

32

EP 0 317 451 A1

**FIG.6**

Office européen
des brevets

**RAPPORT PARTIEL
DE RECHERCHE EUROPEENNE**
qui selon la règle 45 de la Convention sur le brevet
européen est considéré, aux fins de la procédure ultérieure,
comme le rapport de recherche européenne

Numéro de la demande

EP 88 42 0380

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | GB-A- 349 615 (HARRIS) <br> * En entier * | 1-3,5-10,13 | A 61 N 1/32 |
| Y | FR-A-2 502 015 (BENOIT) <br> * En entier * . | 1,3,5-10,13 | |
| Y | FR-A-2 307 554 (LEQUELTE) <br> * En entier * | 1-3,5-10,13 | |
| D,A | US-A-4 406 658 (LATTIN) <br> * Colonne 5, ligne 15 - colonne 8, ligne 66; figure 5 * | 1,2,4,13 | |

--

--

--

----------

**DOMAINES TECHNIQUES
RECHERCHES (Int. Cl 4)**

A 61 N

### RECHERCHE INCOMPLETE

La division de la recherche estime que la présente demande de brevet européen n'est pas conforme aux dispositions de la Convention sur le brevet européen au point qu'une recherche significative sur l'etat de la technique ne peut être effectuée au regard d'une partie des revendications.
Revendications ayant fait l'objet de recherches complètes: 1-6,8-12
Revendications ayant fait l'objet de recherches incomplètes: 7,13
Revendications n'ayant pas fait l'objet de recherches:
Raison pour la limitation de la recherche:

Méthode de traitement chirurgical ou thérapeutique du corps humain ou animal (voir
art. 52(4) de la convention sur le
brevet européen)

| Lieu de la recherche | Date d'achèvement de la recherche | Examinateur |
|---|---|---|
| La Haye | 30-01-1989 | SCHMIERER |